# EUROPEAN PATENT APPLICATION

(11) **EP 1 813 264 A1**
(43) Date of publication of application: **01.08.2007**
(21) Application number: 06001683.9
(22) Date of filing: 27.01.2006
(51) Int. Cl.: A61K 8/81, A61Q 5/10

(54) **Colorants for keratin fibers**

(71) Applicant: Wella Aktiengesellschaft, 64274 Darmstadt (DE)
(72) Inventor: Schmenger, Jürgen, 64331 Weiterstadt (DE); Aeby, Johann, 1723 Marly (CH); Kujawa, Jolanthe, 64289 Darmstadt (DE)
(74) Representative: Bockhorst, Matthias

(57) **Abstract**

The present application relates to an agent for the coloring of keratin fibers based on oxidative dye precursors and/or direct dyes, wherein said agent contains a combination of (i) at least one cationic cellulose derivative and (ii) an acrylamide/ammonium acrylate copolymer and/or Polyacrylate-13 in a suitable cosmetic carrier.

## Description

This invention relates to agents for the coloring of keratin fibers, especially human hair, based on direct dyes and/or oxidative dyes, which contain a mixture of at least one cationic hydroxyethylcellulose and at least one acrylic acid homopolymer or copolymer.

Coloring preparations generally are available in the form of aqueous -preferably thickened- solutions or emulsions and in addition to dyes contain for example fatty alcohols and/or other oil components, emulsifiers and surfactants, or as alcohols if necessary. Oxidative dyes as a rule consist of two components, (i) the dye carrier that contains the dye, and (ii) the oxidant preparation, which shortly before use are mixed together and then applied to the hair that is to be colored. A conditioning step is desirable after rinsing out the oxidative dye. A more or less well-pronounced conditioning effect is obtained whenever a conditioning raw material is added, but by no means can this replace the use of a subsequently applied conditioning agent. Depending on the viscosity and mixture ratio of the two components, a higher or lower viscosity will be obtained upon mixing. Thus, good adhesion of the colorant is achieved especially well through the colorant having a higher viscosity. Moreover, hairdressers often require high viscosity in the materials they use in their work, for example in certain highlighting and lowlighting techniques or foiling techniques as well as for being able to perform spot treatments with a color brush or an accent brush.

The use of cationic hydroxyethylcelluloses in hair dyes is reasonably well known. With regard to their viscosity and adhesion to the hair, however, the conditioning properties and coloring properties of such agents are not satisfactory in all respects. The viscosity of an oxidative dye is raised only marginally by the cellulose derivatives used in the prior art and will possess only an unsatisfactory conditioning effect.

There is thus a significant need for an inexpensive material for the thickening of dye carriers, which will ensure good miscibility of the dye carrier with an oxidizing agent and will provide colorants that have good adhesive properties and coloring properties, and that moreover will exhibit improved conditioning properties in comparison with agents of the prior art.

It has been found that the abovementioned disadvantages of the thickeners used heretofore can be overcome in a superb fashion through the use of a combination of at least one cationic cellulose derivative and certain acrylamide/ammonium acrylate copolymers (CAS-No.: 26100-47-0) and/or Polyacrylate-13 (copolymer from acrylic acid and 2-methyl-2-[(1-oxo-2-propenyl)amino]-1-propanesulfonic acid monosodium salt, 2-propenamide and sodium 2-propenoate; CAS-No.: 15728-72-8), while at the same time excellent conditioning as well as very good viscosity enhancement can be achieved.

The object of the present invention is thus an agent for the coloring of keratin fibers, especially human hair, based on oxidative dye precursors and/or direct dyes (direct dyes means dyes which are able to dye the fibers without tie addition of oxidizing agents or the oxidation by air), wherein said agent contains a combination of (i) at least one cationic cellulose derivative and (ii) an acrylamide/ammonium acrylate copolymer and/or Polyacrylate-13 in a suitable cosmetic carrier.

The cationic cellulose derivative is contained in the colorant of the present invention preferably in a total quantity of from about 0.01 to 20 percent by weight, especially from 0.1 to 5 percent by weight (calculated on the basis of the active substance).

The acrylamide/ammonium acrylate copolymer and/or Polyacrylate-13 is contained in the colorant of the present invention preferably in a total quantity of from about 0.01 to 20 percent by weight, especially from 0.1 to 5 percent by weight (calculated on the basis of the active substance).

For the cationic cellulose derivative, cationic hydroxyethycelluloses (Polyquaternium-10) are especially suitable, such as those for example that are distributed under the trade names Celquat SC-240C (National Starch Co.), Rita Polyquata 400 (Rita Co.), or Ucare Polymer JR types (Amerchol Co.).

The acrylamide/ammonium acrylate copolymer is distributed for example under the trade name Sepiplus 265 from the Seppic Company, while Polyacrylate-13 is distributed for example under the trade name Sepiplus 400 (Seppic Company).

The colorant of the present invention preferably contains oxidative dye precursors, through which the coloring is produced by the action of oxidizing agents, such as for example hydrogen peroxide, or in the presence of atmospheric oxygen (if necessary with the addition of a suitable enzyme system).

Examples that can be named of developer substances and coupler substances as well as self-coupling compounds that are suitable oxidative dye precursors are as follows:
(i) Developer substances: 1,4-diaminobenzene (p-phenylendiamine), 1,4-diamino-2-methylbenzene (p-toluenediamine), 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminoberizene, 1,4-diamino-2-(thiophen-2-yl)benzene, 1,4-diamino-2-(thiophen-3-yl)benzene, 1,4-diamino-2-(pyridin-3-yl)benzene, 2,5-diaminobiphenyl, 1,4-diamino-2-methoxymethylbenzene, 1,4-diamino-2-aminomethylbenzene, 1,4-diamino-2-hydroxymethylbenzene, 1,4-diamino-2-(2-hydroxyethoxy)benzene, 2-(2-(acetylamino)-ethoxy)-1,4-diaminobenzene, 4-phenylaminoaniline, 4-dimethylaminoaniline, 4-diethylaminoaniline, 4-dipropylaminoaniline, 4-[ethyl(2-hydroxyethyl)amino]aniline, 4-[di-(2-hydroxyethyl)amino]aniline, 4-[di-(2-hydroxyethyl)amino]-2-methylaniline, 4-[(2-methoxyethyl)amino]aniline, 4-[(3-hydroxypropyl)amino]aniline, 4-[(2,3-dihydroxypropyl)amino]aniline, 1,4-diamino-2-(2-hydroxyethyl)benzene, 1,4-diamino-2-(1-methylethyl)benzene, 1,3-bis-[(4-aminophenyl)(2-hydroxyethyl)amino]-2-propanol, 1,4-bis-[(4-aminophenyl)amino]butane, 1,8-bis-(2,5-diaminophenoxy)-3,6-dioxaoctane, 4-aminophenol, 4-amino-3-methylphenol, 4-amino-3-(hydroxymethyl)phenol, 4-amino-3-fluorophenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)amino]-methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole, 4,5-diamino-1-methyl-1H-pyrazole, 2-aminophenol, 2-amino-6-methylphenol, 2-amino-5-methylphenol, alone or in mixtures with each other.
(ii) Coupler substances: N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di-[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 2,4-diamino-1,5-di-(2-hydroxyethoxy)-benzene, 1-(2-aminoethoxy)-2,4-diaminobenzene, 2-amino-1-(2-hydroxyethoxy)-4-methylaminobenzene, 2,4-diaminophenoxyacetic acid, 3-[di-(2-hydroxyethyl)amino]-aniline, 4-amino-2-di-[(2-hydroxyethyl)amino]-1-ethoxybenzene, 5-methyl-2-(1-methylethyl)phenol, 3-[(2-hydroxyethyl)amino]aniline, 3-[(2-aminoethyl)-amino]aniline, 1,3-di-(2,4-diaminophenoxy)propane, di-(2,4-diaminophenoxy)methane, 1,3-diamino-2,4-dimethoxybenzene, 2,6-bis-(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 3-amino-2,6-dimethylphenol, 2-methyl-5-(β-hydroxyethylamino)phenol, 5-amino-2-ethylphenol, 2-[(3-hydroxyphenyl)amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]-phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)-amino]-2-methylphenol, 2-amino-3-hydroxypyridine, 5-amino-4-chloro-2-methylphenol, 1-naphthol, 2-methyl-1-naphthol, 1,5-dihydroxy-naphthalene, 1,7-dihydroxynaphthalene, 2,3-dihydroxynaphthalene, 2,7-dihydroxy-naphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole, 2,3-indoline dione, alone or in mixtures with each other.
(iii) Self-coupling compounds: 2-amino-5-methylphenol, 2-amino-6-methylphenol, 2-amino-5-ethoxyphenol, or 2-propylamino-5-aminopyridine.

The total quantity of the oxidative dye precursors contained in the agents of the present invention is up to about 12 percent by weight, especially from about 0.2 to 6 percent by weight.

To obtain specific color shades, moreover, additional conventional natural and/or synthetic direct dyes can be contained in the colorant, for example plant pigments such as henna or indigo, triphenylmethane dyes, aromatic nitro dyes, azo dyes, quinone dyes, cationic dyes (Basic dyes) or anionic dyes (Acid dyes).

Examples of suitable direct dyes that can be used include:
1,4-bis-[(2-hydroxyethyl)amino]-2-nitrobenzene, 1-(2-hydroxyethyl)amino-2-nitro-4-[di-(2-hydroxyethyl)amino]benzene (HC Blue No. 2), 1-amino-3-methyl-4-[(2-hydroxyethyl)amino]-6-nitrobenzene (HC Violet No. 1), 4-[ethyl-(2-hydroxyethyl)-amino]-1-[(2-hydroxyethyl)amino]-2-nitrobenzene hydrochloride (HC Blue No. 12), 4-[di-(2-hydroxyethyl)amino]-1-[(2-methoxyethyl)amino]-2-nitrobenzene (HC Blue No. 11), 1-[(2,3-dihydroxypropyl)amino]-4-[methyl-(2-hydroxyethyl)amino]-2-nitrobenzene (HC Blue No. 10), 1-[(2,3-dihydroxypropyl)amino]-4-[ethyl-(2-hydroxyethyl)amino]-2-nitrobenzene hydrochloride (HC Blue No. 9), 1-(3-hydroxypropylamino)-4-[di-(2-hydroxyethyl)amino]-2-nitrobenzene (HC Violet No. 2), 1-methylamino-4-[methyl-(2,3-dihydroxypropyl)amino]-2-nitrobenzene (HC Blue No. 6), 2-((4-amino-2-nitrophenyl)amino)-5-dimethylaminobenzoic acid (HC Blue No. 13), 1-amino-4-[(2-hydroxyethyl)amino]-2-nitrobenzene (HC Red No. 7), 2-amino-4,6-dinitrophenol, 4-amino-2-nitrodiphenylamine (HC Red No. 1), 1-amino-4-[di-(2-hydroxyethyl)amino]-2-nitrobenzene hydrochloride (HC Red No. 13), 1-amino-5-chloro-4-[(2-hydroxyethyl)-amino]-2-nitrobenzene, 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-[(2-aminoethyl)-amino]-4-(2-hydroxyethoxy)-2-nitrobenzene (HC Orange No. 2), 4-(2,3-dihydroxypropoxy)-1-[(2-hydroxyethyl)amino]-2-nitrobenzene (HC Orange No. 3), 1-amino-5-chloro-4-[(2,3-dihydroxypropyl)amino]-2-nitrobenzene (HC Red No. 10), 5-chloro-1,4-[di-(2,3-dihydroxypropyl)amino]-2-nitrobenzene (HC Red No. 11), 2-[(2-hydroxyethyl)amino]-4,6-dinitrophenol, 4-ethylamino-3-nitrobenzoic acid, 2-[(4-amino-2-nitrophenyl)amino]benzoic acid, 2-chloro-6-ethylamino-4-nitrophenol, 2-amino-6-chloro-4-nitrophenol, 4-[(3-hydroxypropyl)amino]-3-nitrophenol, 2,5-diamino-6-nitropyridine, 1,2,3,4-tetrahydro-6-nitroquinoxaline, 7-amino-3,4-dihydro-6-nitro-2H-1,4-benzoxazine (HC Red No. 14), 1-amino-2-[(2-hydroxyethyl)amino]-5-nitrobenzene (HC Yellow No. 5), 1-(2-hydroxyethoxy)-2-[(2-hydroxyethyl)amino]-5-nitrobenzene (HC Yellow No. 4), 1-[(2-hydroxyethyl)amino]-2-nitrobenzene (HC Yellow No.2),2-[(2-hydroxy-ethyl)amino]-1-methoxy-5-nitrobenzene,2-amino-3-nitrophenol, 1-(2-hydroxyethoxy)-3-methylamino-4-nitrobenzene, 2,3-(dihydroxypropoxy)-3-methylamino-4-nitrobenzene, 2-[(2-hydroxyethyl)amino]-5-nitrophenol (HC Yellow No. 11), 3-[(2-aminoethyl)amino]-1-methoxy-4-nitrobenzene hydrochloride (HC Yellow No. 9), 1-[(2-ureidoethyl)amino]-4-nitrobenzene, 4-[(2,3-dihydroxypropyl)amino]-3-nitro-1-trifluoromethylbenzene (HC Yellow No. 6), 1-chloro-2,4-bis-[(2-hydroxyethyl)amino]-5-nitrobenzene (HC Yellow No. 10), 4-[(2-hydroxyethyl)amino]-3-nitro-1-methylbenzene, 1-chloro-4-[(2-hydroxyethyl)amino]-3-nitrobenzene (HC Yellow No. 12), 4-[(2-hydroxyethyl)amino]-3-nitro-1-trifluoromethylbenzene (HC Yellow No. 13), 4-[(2-hydroxyethyl)amino]-3-nitrobenzonitrile (HC Yellow No. 14), 4-[(2-hydroxyethyl)-amino]-3-nitrobenzamide (HC Yellow No. 15), 1,4-di-[(2,3-dihydroxypropyl)amino]-9,10-anthraquinone, 1-[(2-hydroxyethyl)amino]-4-methylamino-9,10-anthraquinone (CI61505, Disperse Blue No. 3), 2-[(2-aminoethyl)amino]-9,10-anthraquinone (HC Orange No. 5), 1-hydroxy-4-[(4-methyl-2-sulfophenyl)amino]-9, O-anthraquinone, 1-[(3-aminopropyl)amino]-4-methylamino-9,10-anthraquinone (HC Blue No. 8), 1-[(3-aminopropyl)amino]-9,10-anthraquinone (HC Red No. 8), 1,4-diamino-2-methoxy-9,10-anthraquinone (CI62015, Disperse Red No. 11, Solvent Violet No. 26), 1,4-dihydroxy-5,8-bis-[(2-hydroxyethyl)amino]-9,10-anthraquinone (CI62500, Disperse Blue No. 7, Solvent Blue No. 69), 9-(dimethylamino)benzo[a]phenoxazin-7-ium chloride (CI51175; Basic Blue No. 6), 1-((4-amino-3,5-dimethylphenyl)-(2,6-dichlorophenyl)-methylene)-3,5-dimethyl-4-imino-2,5-cyclohexadiene with phosphoric acid (1:1) (Basic Blue 77), di-[4-(diethylamino)phenyl][4-(ethylamino)naphthyl]carbenium chloride (CI42595; Basic Blue No. 7), 3,7-di-(dimethylamino)phenothiazin-5-ium chloride (CI52015; Basic Blue No. 9), di-[4-(dimethylamino)phenyl][4-(phenylamino)naphthyl]-carbenium chloride (CI44045; Basic Blue No. 26), 2-[(4-(ethyl-(2-hydroxyethyl)amino)-phenyl)azo]-6-methoxy-3-methylbenzothiazolium methyl sulfate (CI11154; Basic Blue No. 41), 8-amino-2-bromo-5-hydroxy-4-imino-6-[(3-(trimethylammonio)phenyl)amino]-1(4H)naphthalenone chloride (CI56059; Basic Blue No. 99), bis-[4-(dimethylamino)-plienyl][4-(methylamino)phenyl]carbenium chloride (CI42535; Basic Violet No. 1), tris-[4-(dimethylamino)phenyl]carbenium chloride (CI42555; Basic Violet No. 3), 2-[3,6-(diethylamino)dibenzopyranium-9-yl]benzoic acid chloride (CI45170; Basic Violet No. 10), di-(4-aminophenyl)-(4-amino-3-methylphenyl)-carbenium chloride (CI42510; Basic Violet No. 14), 1,3-bis-[(2,4-diamino-5-methylphenyl)azo]-3-methylbenzene (CI21010; Basic Brown No. 4), 1-[(4-aminophenyl)azo]-7-(trimethylammonio)-2-naphthol chloride (CI12250; Basic Brown No. 16), 1-[(4-amino-2-nitrophenyl)azo]-7-(trimethylammonio)-2-naphthol chloride (Basic Brown No. 17), 1-[(4-amino-3-nitrophenyl)azo]-7-(trimethylammonio)-2-naphthol chloride (CI12251; Basic Brown No. 17), 3,7-diamino-2,8-dimethyl-5-phenylphenazinium chloride (CI50240; Basic Red No. 2), 1,4-dimethyl-5-[(4-(dimethylamino)phenyl)azo]-1,2,4-triazolium chloride (CI11055; Basic Red No. 22), 2-hydroxy-1-[(2-methoxyphenyl)azo]-7-(trimethylammonio)-naphthalene chloride (CI12245; Basic Red No. 76), 2-[2-((2,4-dimethoxyphenyl)amino)-ethenyl]-1,3,3-trimethyl-3H-indol-1-ium chloride (CI48055; Basic Yellow No. 11), 3-methyl-1-phenyl-4-[(3-(trimethylammonio)phenyl)azo]pyrazol-5-one chloride (CI12719; Basic Yellow No. 57), bis-[4-(diethylamino)phenyl]phenylcarbenium bisulfate (1:1) (CI42040; Basic Green No. 1), 1-[di-(2-hydroxyethyl)amino]-3-methyl-4-[(4-nitrophenyl)azo]benzene (CI11210, Disperse Red No. 17), 4-[(4-aminophenyl)azo]-1-[di-(2-hydroxyethyl)amino]-3-methylbenzene (HC Yellow No. 7), 2,6-diamino-3-[(pyridin-3-yl)azo]pyridine, 6-hydroxy-5-[(4-sulfophenyl)azo]-2-naphthalenesulfonic acid disodium salt (CI15985; Food Yellow No. 3; FD&C Yellow No. 6), 2,4-dinitro-1-naphthol-7-sulfonic acid disodium salt (CI10316; Acid Yellow No. 1; Food Yellow No. 1), 2-(indan-1,3-dion-2-yl)quinoline-x,x-sulfonic acid (mixture of mono- and disulfonic acid) (CI47005;D&C Yellow No. 10; Food Yellow No. 13; Acid Yellow No. 3), 5-hydroxy-1-(4-sulfophenyl)-4-[(4-sulfophenyl)azo]pyrazole-3-carboxylic acid trisodium salt (CI19140; Food Yellow No. 4; Acid Yellow No. 23), 9-(2-carboxyphenyl)-6-hydroxy-3H-xanthen-3-one (CI45350; Acid Yellow No. 73; D&C Yellow No. 8), 5-[(2,4-dinitrophenyl)amino]-2-phenylaminobenzenesulfonic acid sodium salt (CI10385; Acid Orange No. 3), 4-[(2,4-dihydroxyphenyl)azo]benzenesulfonic acid monosodium salt (CI14270; Acid Orange No. 6), 4-[(2-hydroxynaphth-1-yl)azo]benzenesulfonic acid sodium salt (CI15510; Acid Orange No. 7), 4-[(2,4-dihydroxy-3-[(2,4-dimethylphenyl)-azo]-phenyl)azo]benzenesulfonic acid sodium salt (CI20170; Acid Orange No. 24), 4-hydroxy-3-[(4-sulfonaphth-1-yl)azo]-1-naphthalenesulfonic acid disodium salt (CI14720; Acid Red No. 14), 6-hydroxy-5-[(4-sulfonaphth-1-yl)azo]-2, 4-naphthalenedisulfonic acid trisodium salt (CI16255; Ponceau 4R; Acid Red No. 18), 3-hydroxy-4-[(4-sulfonaphth-1-yl)azo]-2,7-naphthalenedisulfonic acid trisodium salt (CI16185; Acid Red No. 27), 8-amino-1-hydroxy-2-(phenylazo)-3,6-naphthalenedisulfonic acid disodium salt (CI17200; Acid Red No. 33), 5-(acetylamino)-4-hydroxy-3-[(2-methylphenyl)azo]-2,7-naphthalenedisulfonic acid disodium salt (CI18065; Acid Red No. 35), 2-(3-hydroxy-2,4,5,7-tetraiododibenzopyran-6-on-9-yl)benzoic acid disodium salt (CI45430;Acid Red No. 51), N-[6-(diethylamino)-9-(2,4-disulfophenyl)-3H-xanthen-3-ylidene]-N-ethylethanammonium hydroxide, inner salt, sodium salt (CI45100; Acid Red No. 52), 8-[(4-(phenylazo)phenyl)azo]-7-naphthol-1,3-disulfonic acid disodium salt (CI27290; Acid Red No. 73), 2',4',5',7'-tetrabromo-3',6'-dihydroxyspiro[isobenzofuran-1(3H),9'-[9H]xanthen]-3-one disodium salt (CI45380; Acid Red No. 87), 2',4',5',7'-tetrabromo-4,5,6,7-tetrachloro-3',6'-dihydroxyspiro-[isobenzofuran-1(3H),9'[9H]xanthen]-3-one disodium salt (CI45410; Acid Red No. 92), 3',6'-dihydroxy-4',5'-diiodospiro[isobenzofuran-1(3H),9'(9H)-xanthen]-3-one disodium salt (CI45425; Acid Red No. 95), (2-sulfophenyl)-di-[4-(ethyl((4-sulfophenyl)methyl)-amino)phenyl]-carbenium disodium salt, betaine (CI42090; Acid Blue No. 9; FD&C Blue No. 1), 1,4-bis-[(2-sulfo-4-methylphenyl)amino]-9,10-anthraquinone disodium salt (CI 61570; Acid Green No. 25), bis-[4-(dimethylamino)phenyl]-(3,7-disulfo-2-hydroxynaphth-1-yl)carbenium inner salt, monosodium salt (CI44090; Food Green No. 4; Acid Green No. 50), bis-[4-(diethylamino)phenyl](2,4-disulfophenyl)carbenium inner salt, sodium salt (2:1) (CI42045; Food Blue No. 3; Acid Blue No. 1), bis-[4-(diethylamino)phenyl](5-hydroxy-2,4-disulfophenyl)carbenium inner salt, calcium salt (2:1) (CI42051; Acid Blue No. 3), 1-amino-4-(cyclohexylamino)-9,10-anthraquinone-2-sulfonic acid sodium salt (CI62045; Acid Blue No. 62), 2-(1,3-dihydro-3-oxo-5-sulfo-2H-indol-2-ylidene)-2,3-dihydro-3-oxo-1H-indole-5-sulfonic acid disodium salt (CI73015; Acid Blue No. 74), 9-(2-carboxyphenyl)-3-[(2-methylphenyl)amino]-6-[(2-methyl-4-sulfophenyl)amino]-xanthylium inner salt, monosodium salt (CI45190; Acid Violet No. 9), 1-hydroxy-4-[(4-methyl-2-sulfophenyl)amino]-9,10-anthraquinone sodium salt (CI60730; D&C Violet No. 2; Acid Violet No. 43), bis-[3-nitro-4-[(4-phenylamino)-3-sulfophenylamino]phenyl] sulfone (CI10410; Acid Brown No. 13), 5-amino-4-hydroxy-6-[(4-nitrophenyl)azo]-3-(phenylazo)-2,7-naphthalenedisulfonic acid disodium salt (CI20470; Acid Black No. 1), 3-hydroxy-4-[(2-hydroxynaphth-1-yl)azo]-7-nitro-1-naphthalenesulfonic acid chromium complex (3:2) (CI15711; Acid Black No. 52), 3-[(2,4-dimethyl-5-sulfophenyl)azo]-4-hydroxy-1-naphthalenesulfonic acid disodium salt (CI14700; Food Red No. 1; FD&C Red No. 4), 4-(acetylamino)-5-hydroxy-6-[(7-sulfo-4-[(4-sulfophenyl)azo]naphth-1-yl)azo]-1,7-naphthalenedisulfonic acid tetrasodium salt (CI28440; Food Black No. 1) and 3-hydroxy-4-(3-methyl-5-oxo-1-phenyl-4,5-dihydro-1H-pyrazol-4-ylazo)naphthalene-1-sulfonic acid sodium salt, chromium complex (Acid Red No. 195), alone or in combination with each other.

The total quantity of direct dyes in the agent of the present invention is up to about 7 percent by weight, preferably from about 0.2 to 4 percent by weight.

Further dyes that are conventional and known for hair coloring that can be contained in the colorant of the present invention, among others, are described in E. Sagarin, "Cosmetics, Science and Technology," Interscience Publishers Inc., New York (1957), p. 503 ff. as well as H. Janistyn, "Handbuch der Kosmetika und Riechstoffe," Vol. 3 (1973), p. 388 ff. and K. Schrader "Grundlagen und Rezepturen der Kosmetika," 2nd Ed. (1989), pp. 782-815.

Although oxidative dyes are preferred, it is obviously likewise possible that the colorant of the present invention can be present in the form of a nonoxidative colorant based on the aforementioned direct dyes.

Moreover, the agent of the present invention can contain antioxidants such as for example ascorbic acid, thioglycolic acid or sodium sulfite, as well as chelating agents for heavy metals, for example ethylenediaminetetraacetate or nitriloacetic acid, in an amount of up to about 0.5 percent by weight, or perfume oils in an amount of up to about 1 percent by weight. Obviously, the above-described hair dye can, if necessary, contain further additives that are customary for hair dyes, one example of which is preservatives; another example of which is solvents such as water, lower aliphatic alcohols, for example aliphatic alcohols with from 1 to 4 carbon atoms such as ethanol, propanol and isopropanol, or glycols such as glycerin and 1,2-propylene glycol; or wetting agents or emulsifiers from the classes of anionic, cationic, amphoteric or non-ionogenic surface-active substances; furthermore softening agents; petrolatum; silicone oils, paraffin oil and fatty acids, as well as conditioners, such as cationic resins, lanolin derivatives, cholesterol, vitamins, pantothenic acid and betaine. The abovementioned components are used in the usual amounts for such applications, for example the wetting agents and emulsifiers in concentrations of from 0.1 to 30 percent by weight and the conditioning agents in a concentration of from 0.1 to 5 percent by weight. Especially advantageous in this regard is the addition of nonionic and/or anionic surfactants or emulsifiers, such as for example fatty alcohol sulfates, especially lauryl sulfate and sodium cocoyl sulfate, oxyethylated fatty alcohol sulfates, especially sodium lauryl ether sulfates with from 2 to 4 ethylene oxide units per molecule, oxyethylated fatty acid esters, oxyethylated nonylphenols, oxyethylated fatty alcohols, alkylbenzene sulfonates or fatty acid alkanolamides, in a total quantity of from about 0.1 to 30 percent by weight, preferably 0.2 to 15 percent by weight.

The pH value of the colorant of the present invention for nonoxidative colorants based on direct dyes will be in the range of from about 5 to 10, preferably from 6 to 9, while for oxidative colorants based on oxidative dye precursors the pH value will be in the range of from about 6 to 12, preferably from 8 to 11, where the pH value of the ready-to-use oxidative hair colorant (in other words, the mixture of the hair dye of the present invention with the oxidizing agent) will be from about 5.5 to 10, preferably from 6 to 9.

Depending on the composition and desired pH value of the colorant, the adjustment of the pH value is preferably carried out with ammonia or organic amines, such as for example glucamines, aminomethylpropanol, monoethanolamine or triethanolamine, inorganic bases, for example sodium hydroxide, potassium hydroxide, sodium carbonate or calcium hydroxide, or respectively with organic or inorganic acids, such as for example lactic acid, citric acid, acetic acid or phosphoric acid.

The agent of the present invention is preferably formulated as an aqueous or aqueous alcoholic preparation, for example as a thickened solution, as an emulsion, as a creme or as a gel. The colorant can also be present in the form of a two-component agent, in which the dye is separated from the dye carrier mass and the two components are mixed together immediately before use to provide an agent of the present invention.

The compositions according to the present invention may comprise at least one source of an oxidizing agent for developing the hair color. Preferred oxidizing agents for use herein are water-soluble peroxygen oxidizing agents. "Water-soluble" as defined herein means that in standard condition at least 0.1g, preferably 1g, more preferably 10g of said oxidizing agent can be dissolved in 1 liter of deionized water. The oxidizing agents are valuable for the initial solubilisation and decolourisation of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is dissolved prior to use. Preferred for use in the compositions according to the present invention are hydrogen peroxide, percarbonate, persulphates and combinations thereof.

According to the present invention the compositions comprise from about 0.1 % to about 15% by weight, preferably from about 1% to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably 7.5 to 9.5 more preferably about pH 9. Moreover, this system is also particularly effective in combination with a source of ammonia or ammonium ions. It has been found that this oxidizing agent can deliver improvements to the desired hair colour results particularly with regard to the delivery of high lift, whilst considerably reducing the odour, skin and scalp irritation and damage to the hair fibres.

Accordingly, any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the compositions comprise from about 0.1% to about 15% by weight, preferably from about 1% to about 10% by weight, and most preferably from about 1% to about 8% by weight of a hydrogencarbonate ion and from about 0.1 % to about 10% by weight, preferably from about 1% to about 7% by weight, and most preferably from about 2% to about 5% by weight of a source of hydrogen peroxide.

Especially preferred oxidants for developing the hair color are mainly hydrogen peroxide or a compound of addition of hydrogen peroxide to urea, melamine, sodium borate or sodium carbonate, in the form of a 3 to 12%, preferably 6%, aqueous solution, as well as air oxygen. When a 6% hydrogen peroxide solution is used as the oxidant, the weight ratio of hair colorant to oxidant is 5:1 to 2:1, and preferably 1:1. Larger amounts of oxidant are used primarily when the hair colorant contains a higher dye concentration or when stronger hair bleaching is desired at the same time.

According to the present invention the compositions may further comprise a source of radical scavenger. As used herein the term radical scavenger refers to a species that can react with a reactive radical, preferably carbonate radicals, to convert the reactive radical by a series of fast reactions to a less reactive species. Suitable radical scavengers for use herein include compounds according to the general formula (RSI):

R¹-Y-C(H)(R³)-R⁴-(C(H)(R⁵)-Y-R⁶)ₙ (RSI)

wherein Y is NR², O, or S, preferably NR², n is 0 to 2, and wherein R⁴ is monovalent or divalent and is selected from: (a) substituted or unsubstituted, straight or branched, alkyl, mono- or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein R⁴ can be connected to R³ or R⁵ to create a 5, 6 or 7 membered ring; and wherein R¹, R², R³, R⁵, and R⁶ are monovalent and are selected independently from: (a), (b) and (c) described herein above, or H.

Preferably, R⁴ is selected from: (a) substituted or unsubstituted, straight or branched, alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; more preferably R⁴ is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.

Preferably, the R⁴ systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; O being paricularly preferred.

Preferably, R¹, R², R³, R⁵, and R⁶ are selected independently from any of the systems defined for R⁴ above, and H.

In alternative embodiments, any of R¹, R², R³, R⁴, R⁵, and R⁶ groups are substituted. Preferably, the substituent(s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, monofluoroalkyl, polyfluoroalkyl or perfluoroalkyl systems; said systems of (i), (ii) and (iii) comprising from 1 to 10 carbon atoms and 0 to 5 heteroatoms selected from O, S, N, P, and Si; (b) the group of S-linked monovalent substituents consisting of SA¹, SCN, SO₂A¹, SO₃A¹, SSA¹, SOA¹, SO₂NA¹A², SNA¹A², and SONA¹A²; (c) the group of O-linked monovalent substituents consisting of OA¹, OCN and ONA¹A²; (d) the group ofN-linked monovalent substituents consisting of NA¹A², (NA¹A²A³)⁺, NC, NA¹OA², NA¹SA², NCO, NCS, NO₂, N=NA¹, N=NOA¹, NA¹CN, NA¹NA²A³; (e) the group of monovalent substituents consisting of COOA¹, CON₃, CONA¹₂, CONA¹COA², C(=NA¹)NA¹A², CHO, CHS, CN, NC, and X; and (f) the group consisting fluoroalkyl monovalent substituents consisting of monofluoroalkyl, polyfluoroalkyl perfluoroalkyl systems comprising from 1 to 12 carbon atoms and 0 to 4 heteroatoms.

For the groups (b) to (e), described above, A¹, A², and A³ are monovalent and are independently selected from: (1) H, (2) substituted or unsubstituted, straight or branched, alkyl, monounsaturated or poly-unsaturated alkyl, heteroalkyl, aliphatic, heteroaliphatic or heteroolefinic systems, (3) substituted or unsubstituted, monocyclic or polycyclic aliphatic, aryl or heterocyclic systems, or (4) substituted or unsubstituted, monofluoroalkyl, polyfluoroalkyl or perfluoroalkyl systems; said systems of (2), (3) and (4) comprising from 1 to 10 carbon atoms and 0 to 5 heteroatoms selected from O, S, N,P, and Si; and wherein X is a halogen selected from the group consisting of F, Cl, Br, and I.

Preferred substituents for use herein include those having a Hammett Sigma Para (σₚ) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375).

Alternative suitable radical scavengers for use herein are compounds according to the general formula (RSII) : 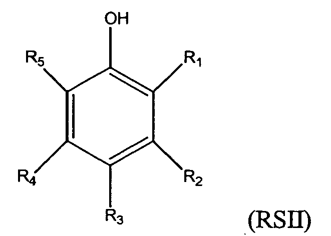 wherein R₁, R₂, R₃, R₄, and R₅ are each independently selected from H, COO⁻M⁺, Cl, Br, SO₃⁻M⁺, NO₂, OCH₃, OH or a C¹ to C¹⁰ primary or secondary alkyl and M is either H or alkali metal. Preferably, the above-described radical scavengers have a pKa of more than 8.5 to ensure protonation of the hydroxy goup.

Other suitable radical scavengers for use herein include those selected from group (RSIII) benzylamine, imidazole, di-tert-butylhydroxytoluene, hydroquinone, guanine, pyrazine, piperidine, morpholine, methylmorpholine, 2-methyoxyethylamine, and mixtures thereof.

Preferred radical scavengers according to the present invention are selected from the classes of alkanolamines, amino sugars, amino acids, esters of amino acids and mixtures thereof. Particularly preferred compounds are: monoethanolamine, 3-amino-1-propanol, 4-amino-1-butanol, 5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof.

The radical scavengers according to the present invention preferably have a molecular weight of less than about 500, preferably less than about 300, more preferably less than about 250 in order to facilitate penetration of the radical scavenger into the hair fibre. The compositions of the present invention preferably comprise from about 0.1% to about 10% by weight, preferably from about 1% to about 7% by weight of radical scavenger. The radical scavenger is also preferably selected such that it is not an identical species as the alkalizing agent. According to one embodiment of the present invention the radical scavenger may be formed insitu in the hair dyeing compositions prior to application to the hair fibres.

To use the afore-described colorants for oxidative dyeing of hair, said colorants are mixed with an oxidant immediately before use, and the mixture is applied to hair in an amount sufficient for hair treatment which, depending on hair fullness, is generally from about 60 to 200 grams.

Provided that the colorant of the present invention contains no oxidative dye precursors or contains such oxidative dye precursors that are easily oxidizable with atmospheric oxygen (with the addition of a suitable enzyme system if necessary), it can be applied directly to the keratin fibers without being mixed with an oxidizing agent beforehand.

Oxidizing agents that can be used for developing the coloring include especially hydrogen peroxide or its addition compounds with urea, melanin or sodium borate in the form of a 1 to 12% aqueous solution, and preferably a 1.5 to 6% aqueous solution. The mixture ratio of colorant to oxidizing agent depends on the concentration of the oxidizing agent and will generally be from about 5:1 to 1:2, preferably 1:1, where the content of the oxidizing agent in the ready-to-use preparation is preferably from about 0.5 to 8 percent by weight, especially from 1 to 4 percent by weight.

The ready-to-use colorant is allowed to act on the keratin fibers (for example human hair) at a temperature of from 15 °C to 50 °C for a period of from about 10 to 45 minutes, preferably from about 15 to 30 minutes, then the fibers are rinsed with water and dried. If necessary, the fibers can be washed with a shampoo in connection with this rinsing, and possibly post-rinsed with a weak organic acid, such as for example tartaric acid. The keratin fibers are subsequently dried.

If necessary, the viscosity of the oxidative dye of the present invention can obviously also be raised after mixing with the oxidizing agent through the subsequent addition of a thickener combination of the present invention, which enables simpler and more inexpensive base formulations.

The colorant of the present invention fulfills the stated requirements in an outstanding manner with respect to the adhesive properties, the conditioning properties, the application behavior and the adjustment of the viscosity, and are noticeably easier to apply than comparable agents. Furthermore, the colorants of the present invention possess a uniform consistency and an attractive cosmetic appearance. Of particular note is the very good viscosity and the outstanding stability of the agents of the present invention, as well as their excellent adhesion to hair. Furthermore, the application of the agent of the present invention enables a variation of the weight ratio of colorant to oxidizing agent over a broad range (for example from 1:1 to 1:3) without any noticeable adverse effects on the viscosity or on the adhesion properties of the ready-to-use oxidative dye.

A further object of the present invention is a preparation that contains an oxidizing agent, for example hydrogen peroxide or its addition compounds with urea, melanin or sodium borate in the form of a 1-12%, and preferably a 1.5-6% aqueous solution, as well as a combination of (i) at least one cationic cellulose derivative and (ii) an acrylamide/ammonium acrylate copolymer and/or Polyacrylate-13.

Another object of the present invention is a preparation for the thickening of cosmetic agents, especially hair dyes, which contains a combination of (i) at least one cationic cellulose derivative and (ii) an acrylamide/ammonium acrylate copolymer and/or Polyacrylate-13.

Yet another object of the present invention is a two-component kit, the two components of which are mixed together before use, where said kit comprises a dye carrier mass (A) that contains an oxidative dye precursor and/or a direct dye and at least one cationic cellulose derivative, and an oxidant preparation (B) that contains an oxidizing agent and an acrylamide/ammonium acrylate copolymer and/or Polyacrylate 13.

A further object of the present invention is a two-component kit, the two components of which are mixed together before use, where said kit comprises a dye carrier mass (A) that contains oxidative dye precursors and/or direct dyes and an acrylamide/ammonium acrylate copolymer and/or Polyacrylate-13, and an oxidative preparation (B) that contains an oxidizing agent and at least one cationic cellulose derivative.

The following Examples should serve to illustrate the object of the invention in more detail, without the invention being limited to these examples.

### Examples

### Example 1: Creme-type oxidative hair colorant

### Component A: Base creme without dyes

| | |
|---|---|
| 7.00 g | cetyl stearyl alcohol |
| 3.00 g | glycol distearate |
| 3.00 g | polyethylene glycol (25) cetyl stearyl ether |
| 9.00 g | coco fatty acid monoethanolamide |
| 3.00 g | polyethylene glycol (7) glyceryl monococoate |
| 4.00 g | lauryl ether sulfate |
| 0.50 g | acrylamide/ammonium acrylate copolymer (Sepiplus 265 from the Seppic Company) |
| 0.30 g | Polyquaternium 10 (O-[2-hydroxy-3-(trimethylammonio)-propyl]hydroxyethylcellulose chloride) |
| 0.10 g | ethylenediamineacetic acid |
| 0.20 g | ascorbic acid |
| 0.40 g | sodium sulfite |
| 0.25 g | perfume |
| 9.50 g | ammonia, 25% aqueous solution |
| balance to 100.00 g | water, completely desalinated |

### Component (B): Dye in powder or granular form

| | |
|---|---|
| 6.810 g | 4-aminophenol |
| 2.500 g | 1-naphthol |
| 0.068 g | resorcinol |
| 0.017 g | 2-amino-6-chloro-4-nitrophenol |

50 g of the abovementioned base creme (A) were mixed immediately before use with 10 g of the abovementioned dye powder or granules (B) and 50 g of a 6% aqueous hydrogen peroxide solution. A homogeneous, cosmetically attractive, optimally thickened coloring preparation was obtained. The mixture so obtained was then applied to medium blonde natural hair. After an action period of 30 minutes at 40 °C, the hair was rinsed with water and dried. The hair obtained a bright, reddish-brown color.

### Example 2: Oxidative hair colorant, liquid

| | |
|---|---|
| 0.3000 g | Dow Coming Softcat SL-60 (cationic cellulose ether; Polyquaternium-67) |
| 0.4000 g | Polyacrylate-13 (Sepiplus 400 from the Seppic Company) |
| 5.0000 g | 2-octyl-1-dodecanol |
| 15.0000 g | oleic acid |
| 10.0000 g | sodium lauryl alcohol diglycol ether sulfate (28% aqueous solution) |
| 1.3620 g | 4-aminophenol |
| 0.5000 g | 1-naphthol |
| 0.0136 g | resorcinol |
| 0.0034 g | 2-amino-6-chloro-4-nitrophenol |
| 12.0000 g | ammonia, 25% aqueous solution |
| 1.0000 g | ethylenediaminetetraacetic acid disodium salt |
| 1.0000 g | ascorbic acid |
| 15.0000 g | isopropanol |
| balance to 100.0000 g | water |

50 g of the abovementioned hair colorant was mixed immediately before use with 50 g of a 6% aqueous hydrogen peroxide solution. A homogeneous, cosmetically attractive, optimally thickened coloring preparation was obtained. The mixture so obtained was then applied to blonde natural hair. After an action period of 30 minutes at 40 °C, the hair was rinsed with water and dried. The hair obtained a bright, copper-red color.

### Example 3: Gel-type oxidative hair colorant for brilliant coloring

### Component (A): Liquid dye carrier mass

| | |
|---|---|
| 0.70 g | Polyacrylate-13 (Sepiplus 400 from the Seppic Company) |
| 0.25 g | Polyquaternium-10 (O-[2-hydroxy-3-(trimethylammonio)-propyl]hydroxyethylcellulose chloride) |
| 10.00 g | lauryl alcohol |
| 6.00 g | nonylphenol, oxyethylated with 4 moles of ethylene oxide |
| 6.00 g | oleic acid |
| 0.50 g | p-phenylenediamine |
| 0.07 g | resorcinol |
| 5.00 g | sodium lauryl alcohol diglycol ether sulfate, 28% aqueous solution |
| 1.00 g | ethylenediaminetetraacetic acid-disodium salt |
| 18.00 g | ammonia, 25% aqueous solution |
| 8.00 g | ethanol |
| balance to 100.00 g | water |

### Component (B): Hydrogen peroxide emulsion

| | |
|---|---|
| 10.0 g | cetyl stearyl alcohol |
| 1.5 g | cholesterol |
| 4.0 g | sodium lauryl alcohol diglycol ether sulfate, 28% aqueous solution |
| 35.0 g | hydrogen peroxide, 35% aqueous solution |
| 0.3 g | perfume |
| balance to 100.0 g | water |

Before use, 40 g of the liquid dye carrier mass (A) were mixed with 80 g of the hydrogen peroxide emulsion (B), corresponding to a mixture ratio of (A):(B) ~ 1:2, and 120 g of this mixture was applied to gray human hair. After an action period of 20 minutes at room temperature the hair was rinsed with water and dried. The hair treated in this manner was uniformly colored light brown from the hair roots to the hair tips. The agent of the present invention was easily applied and did not run from the hair.

### Example 4: Oxidative hair colorant, creme-type

| | |
|---|---|
| 0.40 g | acrylamide/ammonium acrylate copolymer (Sepiplus 265 from the Seppic Company) |
| 0.40 g | Polyquaternium-10 (O-[2-hydroxy-3-(trimethylammonio)-propyl]hydroxyethylcellulose chloride) |
| 3.00 g | oleyl alcohol |
| 15.00 g | cetyl alcohol |
| 3.50 g | sodium lauryl alcohol diglycol ether sulfate (28% aqueous solution) |
| 3.00 g | monoethanolamine |
| 1.30 g | 1-methyl-2,5-diaminobenzene |
| 1.00 g | beeswax |
| 0.65 g | resorcinol |
| 0.50 g | keratin hydrolysate |
| 0.50 g | silk protein hydrolysate |
| 0.50 g | 2-amino-6-chloro-4-nitrophenol |
| 0.30 g | ascorbic acid |
| balance to 100.00 g | water |

50 g of the abovementioned hair colorant were mixed immediately before use with 50 g of a 12% aqueous hydrogen peroxide solution. The mixture so obtained was then applied to blonde natural hair. After an action period of 30 minutes at 40 °C, the hair was rinsed with water and dried. A uniform, robust brown tint was obtained.

### Example 5: Non-oxidative hair tint

| | |
|---|---|
| 2.50 g | Polyacrylate-13 (Sepiplus 400 from the Seppic Company) |
| 0.25 g | Polyquaternium-10 (O-[2-hydroxy-3-(trimethylammonio)-propyl]hydroxyethylcellulose chloride) |
| 6.00 g | lauryl alcohol |
| 5.00 g | sodium lauryl sulfate |
| 3.00 g | cetyl stearyl alcohol and sodium lauryl sulfate (Lanette W) |
| 1.50 g | 2-amino-6-chloro-4-nitrophenol |
| 1.00 g | monoethanolamine |
| 1.00 g | beeswax |
| 0.50 g | keratin hydrolysate |
| 0.30 g | silk protein hydrolysate |
| 0.20 g | glycine |
| balance to 100.00 g | water |

A slightly gel-like coloring mass was obtained that could be applied easily and uniformly, and also adhered well to the hair as a result of its outstanding viscosity properties. After an action period of 20 minutes at 20 °C, the hair was rinsed with lukewarm water, set in a style and dried. The hair treated in this way had a uniform, robust, bright golden-orange color.

### Example 6: Oxidative hair colorant, creme-type

### Component (A): Dye carrier mass

| | |
|---|---|
| 1.5 g | acrylamide/ammonium acrylate copolymer (Sepiplus 265 from the Seppic Company) |
| 0.3 g | Polyquaternium 10 (O-[2-hydroxy-3-(trimethylammonio)-propyl]hydroxyethylcellulose chloride) |
| 8.0 g | 2-octyl-1-dodecanol |
| 3.0 g | sodium lauryl alcohol diglycol ether sulfate, 28% aqueous solution |
| 2.8 g | 2,5-diaminotoluene sulfate |
| 1.0 g | resorcinol |
| 0.4 g | m-aminophenol |
| 0.2 g | 2-amino-4-(2'-hydroxyethylamino)anisole sulfate |
| 0.3 g | ascorbic acid |
| 0.1 g | ethylenediaminetetraacetic acid |
| 12.2 g | ammonia, 25% aqueous solution |
| 2.0 g | ethanol |
| balance to 100.0 g | water |

### Component (B): Hydrogen peroxide - emulsion

| | |
|---|---|
| 10.0 g | cetyl stearyl alcohol |
| 1.5 g | cholesterol |
| 4.0 g | sodium laury alcohol diglycol ether sulfate, 28% aqueous solution |
| 17.0 g | hydrogen peroxide, 35% aqueous solution |
| 0.3 g | perfume |
| balance to 100.0 g | water |

Before use, 40 g of the liquid dye carrier mass (A) were mixed with 80 g of the hydrogen peroxide emulsion (B), corresponding to a mixture ratio of (A):(B) ~ 1:2, and 120 g of this mixture was applied to gray human hair. After an action period of 20 minutes at room temperature, the hair with was rinsed with water and dried. The hair treated in this manner had obtained a uniform, dark brown tint. This agent of the present invention adhered very well to the hair without running off.

### Example 7: Multicomponent oxidative hair colorant

### Component (A): Dye carrier mass

| | |
|---|---|
| 0.75 g | Polyacrylate-13 (Sepiplus 400 from the Seppic Company) |
| 0.30 g | Polyquaternium-10 (O-[2-hydroxy-3-(trimethylammonio)-propyl]hydroxyethylcellulose chloride) |
| 8.00 g | 2-octyl-1-dodecanol |
| 9.00 g | cetyl stearyl alcohol |
| 3.00 g | sodium lauryl alcohol diglycol ether sulfate, 28% aqueous solution |
| 0.30 g | ascorbic acid |
| 0.10 g | ethylenediaminetetraacetic acid |
| 12.20 g | ammonia, 25% aqueous solution |
| 2.00 g | ethanol |
| balance to 100.00 g | water |

The dyes were added in powder or granular form. Examples of granular base shades:

| BASE SHADES: | yellow | golden | orange | red | purple | pink | blue | brown | green |
|---|---|---|---|---|---|---|---|---|---|
| 2,5-Diaminotoluene sulfate | | | | | | | | | 4.95 |
| 2,4-Diaminophenoxyethanol*HCl | | | | | | | 3.86 | | |
| p-Phenylenediamine | | | | | | | | 2.40 | |
| p-Aminophenol | | | 1.50 | | | | | | |
| Resorcinol | | | | | | | | 1.80 | |
| m-Aminophenol | | | | | | | | 0.64 | |
| 4-Amino-2-hydroxytoluene | | | 2.05 | 1.12 | 1.17 | | | | |
| 1-Naphthol | | | | | | 1.73 | | | |
| 6-Amino-m-cresol | 1.50 | | | | | | | | |
| Hydroxyethyl-3,4-methylenedioxyaniline *HCl | | | | | | | | | 4.90 |
| N,N-bis-(2-Hydroxyethyl)-p-phenylenediamine sulfate | | | | | 2.80 | | 4.71 | | |
| 1-Hydroxyethyl-4,5-diaminopyrazole sulfate | | | | 2.18 | | 2.88 | | | |
| 2-Amino-6-chloro-4-nitrophenol | | 2.00 | | | | | | | |
| N-(2-Hydroxyethyl)-2-nitro-4-trifluoromethylaniline | | | | | | | | | 3.00 |

The base shades can optionally be mixed with each other to achieve a wide variety of different colors.

### Component B: Hydrogen peroxide emulsion

| | |
|---|---|
| 10.0 g | cetyl stearyl alcohol |
| 1.5 g | cholesterol |
| 4.0 g | sodium lauryl alcohol diglycol ether sulfate, 28% aqueous solution |
| 35.0 g | hydrogen peroxide, 35% aqueous solution |
| 0.3 g | perfume |
| balance to 100.0 g | water |

Before use, 40 g of the dye carrier mass (A) were mixed with 80 g of the hydrogen peroxide emulsion (B), corresponding to a mixture ratio of (A):(B) ~ 1:2, to which mixtures of granular base tints were added, the entire preparation was stirred and then 120 g of this mixture was applied to gray human hair. After an action period of 20 minutes at room temperature, the hair was rinsed with water and dried. The hair treated in this manner had obtained a tint depending on the mixture of base tints. This agent of the present invention adhered well to the hair without running off.

### Example 8: Two-component oxidative hair colorant

### Component (A): Dye carrier mass

| | |
|---|---|
| 8.0 g | 2-octyl-1-dodecanol |
| 3.0 g | sodium lauryl alcohol diglycol ether sulfate, 28% aqueous solution |
| 2.8 g | 2,5-diaminotoluene sulfate |
| 1.0 g | resorcinol |
| 0.4 g | m-aminophenol |
| 0.2 g | 2-amino-4-(2'-hydroxyethylamino)anisole sulfate |
| 0.3 g | ascorbic acid |
| 0.1 g | ethylenediaminetetraacetic acid |
| 12.2 g | ammonia, 25% aqueous solution |
| 0.3 g | Polyquaternium-10 (0-[2-hydroxy-3-(trimethylammonio)-propyl]hydroxyethylcellulose chloride) |
| 2.0 g | ethanol |
| balance to 100.0 g | water |

### Component (B): Hydrogen peroxide emulsion

| | |
|---|---|
| 1.2 g | Polyacrylate-13 (Sepiplus 400 from the Seppic Company) |
| 10.0 g | cetyl stearyl alcohol |
| 1.5 g | cholesterol |
| 4.0 g | sodium lauryl alcohol diglycol ether sulfate, 28% aqueous solution |
| 25.7 g | hydrogen peroxide, 35% aqueous solution |
| 0.3 g | perfume |
| balance to 100.0 g | water |

Before use, 40 g of the dye carrier mass (A) was mixed with 80 g of the hydrogen peroxide emulsion (B), corresponding to a mixture ratio of (A):(B) ~ 1:2, and 120 g of this mixture were applied to gray human hair. After an action period of 20 minutes at room temperature, the hair was rinsed with water and dried. The hair treated in this manner had obtained a uniform, dark brown tint. This agent of the present invention adhered well to the hair without running off.

### Example 9: Use of Color thickener

### Component A: Liquid dye carrier mass

| | |
|---|---|
| 10.00 g | lauryl alcohol |
| 6.00 g | nonylphenol, oxyethylated with 4 moles of ethylene oxide |
| 6.00 g | oleic acid |
| 0.50 g | p-phenylenediamine |
| 0.07 g | resorcinol |
| 5.00 g | sodium lauryl alcohol diglycol ether sulfate, 28% aqueous solution |
| 1.00 g | ethylenediaminetetraacetic acid-disodium salt |
| 18.00 g | ammonia, 25% aqueous solution |
| 8.00 g | ethanol |
| balance to 100.00 g | water |

### Component B: Hydrogen peroxide emulsion

| | |
|---|---|
| 10.0 g | cetyl stearyl alcohol |
| 1.5 g | cholesterol |
| 4.0 g | sodium lauryl alcohol diglycol ether sulfate, 28% aqueous solution |
| 35.0 g | hydrogen peroxide, 35% aqueous solution |
| 0.3 g | perfume |
| balance to 100.0 g | water |

### Component C: Color thickener

| | |
|---|---|
| 10.0 g | acrylamide/ammonium acrylate copolymer (Sepiplus 265 from the Seppic Company) |
| 3.0 g | Polyquaternium-10 (O-[2-hydroxy-3-(trimethylammonio)-propyl]hydroxyethylcellulose chloride) |
| 0.3 g | perfume |
| 10.0 g | ethanol |
| balance to 100.0 g | water |

Before use, 40 g of the liquid dye carrier mass (A) were mixed with 40 g of the hydrogen peroxide emulsion (B), corresponding to a mixture ratio of (A):(B) ~ 1:1, and the color thickener (C) was added to this to achieve the desired viscosity (from about 1 g to 3 g), and the mixture obtained was applied to gray human hair. After an action period of 20 minutes at room temperature, the hair was rinsed with water and dried. The hair treated in this manner had obtained a uniform dark brown tint. This agent of the present invention adhered well to the hair without running off.

Unless indicated otherwise, all percentages given in the present application represent weight percents.

## Claims

1. An agent for the coloring of keratin fibers based on oxidative dye precursors and/or direct dyes, **characterized in that** said agent contains a combination of (i) at least one cationic cellulose derivative and (ii) an acrylamide/ammonium acrylate copolymer and/or Polyacrylate 13 in a suitable cosmetic carrier.

2. The agent according to Claim 1, **characterized in that** the cationic cellulose derivative is Polyquaternium-10.

3. The agent according to Claim 1 or 2, **characterized in that** the cationic cellulose derivative is contained in a total quantity of from 0.01 to 20 percent by weight.

4. The agent according to one of Claims 1 to 3, **characterized in that** the acrylamide/ammonium acrylate copolymer and/or Polyacrylate-13 is/are contained in a total quantity of from 0.01 to 20 percent by weight.

5. The agent according to one Claims 1 to 4, **characterized in that** said agent contains oxidative dye precursors in a total quantity of up to 12 percent by weight.

6. The agent according to one of Claims 1 to 5, **characterized in that** said agent contains direct dyes in a total quantity of up to 7 percent by weight.

7. The agent according to one of Claims 1 to 6, **characterized in that** said agent is a hair colorant.

8. The agent according to one of Claims 1 to 7, **characterized in that** said agent is mixed with an oxidizing agent prior to use.

9. A preparation containing an oxidizing agent as well as a combination of (i) at least one cationic cellulose derivative and (ii) an acrylamide/ammonium acrylate copolymer and/or Polyacrylate-13.

10. A preparation for the thickening of cosmetic agents, **characterized in that** said preparation contains a combination of (i) at least one cationic cellulose derivative and (ii) an acrylamide/ammonium acrylate copolymer and/or Polyacrylate-13.

11. A two-component kit, the two components of which are mixed with each other prior to use, comprising a dye carrier mass (A) that contains oxidative dye precursors and/or direct-penetrating dyes and at least one cationic cellulose derivative, and an oxidant preparation (B) that contains an oxidizing agent and an acrylamide/ammonium acrylate copolymer and/or Polyacrylate-13.

12. A two-component kit, the two components of which are mixed with each other prior to use, comprising a dye carrier mass (A) that contains oxidative dye precursors and/or direct dyes and an acrylamide/ammonium acrylate copolymer and/or Polyacrylate-13, and an oxidant preparation (B) that contains an oxidizing agent and at least one cationic cellulose derivative.

13. A ready-to-use agent for the coloring of hair that contains oxidative dye precursors and/or direct dyes, and an oxidizing agent as well as a combination of (i) at least one cationic cellulose derivative and (ii) an acrylamide/ammonium acrylate copolymer and/or Polyacrylate-13.
